# EUROPEAN PATENT SPECIFICATION

(11) **EP 0 713 713 B1**
(45) Date of publication and mention of the grant of the patent: **21.06.2000**
(21) Application number: 95203145.8
(22) Date of filing: 16.11.1995
(51) Int. Cl.: A61M 29/00

(54) **Balloon catheter**
Ballonkatheter
Cathéter à ballonet

(30) Priority: 22.11.1994 NL 9401951
(43) Date of publication of application: 29.05.1996
(73) Proprietor: CORDIS EUROPA N.V., NL-9301 LJ Roden (NL)
(72) Inventor: Mulder, Hugo, NL-9737 NT Groningen (NL)
(74) Representative: 't Jong, Bastiaan Jacobus

(56) References cited:
- WO-A-83/03204
- GB-A- 2 020 557

## Description

The invention relates to a balloon catheter comprising a tube-like basic body with a proximal and a distal end. A balloon-shaped member has been arranged at the distal end, and this balloon-shaped member can be expanded by supplying medium under pressure to it via a lumen in the basic body. At both ends the balloon member is connected with the basic body in a sealed manner. In a section enclosed by the balloon member and positioned in between these sealed connections, a number of openings have been arranged in the wall of the basic body. WO-A-83/03204 describes a catheter comprising two sections of catheter tube connected by a rigid balloon support. The balloon surrounds the rigid support, the latter being provided with longitudinal openings in order to be able to supply the medium under pressure from the lumen to the inside of the balloon member and to remove it from there.

The expansion possibilities of the balloon are limited by the fact that an expansion in radial direction has to be accompanied by a reduction in axial direction. The ends of the balloon member are however fixed to the basic body, so that with the catheters manufactured according to the current state of the technique, axial reduction is not possible.

With the catheter according to the invention a number of elongated openings, extending in a longitudinal direction, have been arranged in the wall of the section of the basic body enclosed by the balloon member. In between these openings strip-shaped elements, which can bend outwards, remain. Due to this bending outwards the enclosed section can be reduced axially, as a result of which the balloon member can expand freely. On supplying medium under pressure an axial compressive force is exerted on the enclosed section due to the radial expansion, whereby the ends thereof are forced towards each other and, as a result of this compressive force, the strip-shaped sections will bend outwards from in between the openings.

In the non-expanded state of the balloon the strip-shaped parts in between the openings are straight, as a result of which they can withstand sufficient compressive force which is generated when introducing a balloon catheter into a patient.

By employing the measures according to the invention the balloon member can, in non-expended state, have a small diameter and can be positioned tightly against the basic body. In the expanded state such a balloon can have a large effective diameter.

In order to obtain a suitable bending performance the measure as set out in claim 2 is preferably employed. The strip- shaped wall sections bend outwards uniformly around the entire circumference, as a result of which the longitudinal axis of the balloon remains in line with the catheter. The balloon will not be pulled out of position on expansion.

According to a further development the measure as set out in claim 3 is employed. Consequently the entire axial reduction is spread over the different groups, so that the strip-shaped sections in between each group of openings need to be subjected to only relatively little deformation and do not bend outwards too much.

A suitable embodiment is characterised in claim 4. In this way sufficient deformation is combined with a suitable stiffness. By employing the measure as set out in claim 5, the cross-section of the strip-shaped sections becomes practically flat, so that they bend outwards easily and spring back into their original shape again.

The catheter according to the invention is preferably manufactured as characterised in claim 6.

In case the catheter according to the invention is provided with a tube-like element which extends inside the lumen of the basic body, the intended use of which tube-like element is for instance supplying contrast medium to the distal end of the catheter, the measure as set out in claim 7 is preferably employed.

The invention can be employed particularly well with the measure as set out in claim 8. The shape of the expanded balloon can be determined carefully in advance, while the significant axial reduction occurring during expansion can be absorbed well.

The invention will be explained in greater detail in the following description with reference to the attached drawings.

Figure 1 shows a distal end-section of the catheter according to the invention with the balloon member in non- expanded state.

Figure 2 shows a view corresponding to that of figure 1 of the distal end-section of the catheter of figure 1 with the balloon in expanded state of use.

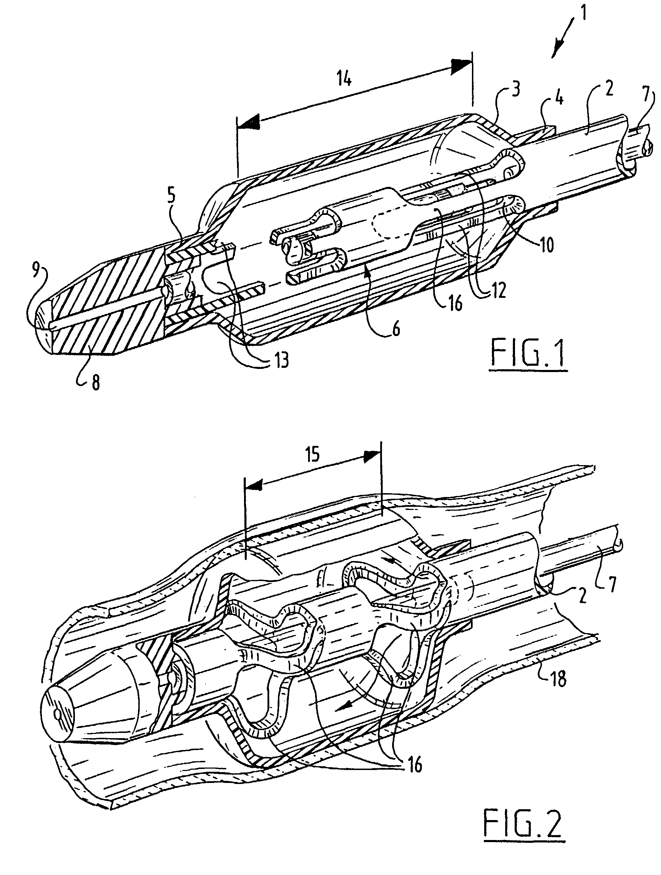

The distal end-section of a catheter 1 illustrated in fig. 1 shows the basic body 2 of that catheter on which a balloon-shaped element 3 has been arranged. At the right end 4 and left end 5 as seen in fig. 1, the balloon member 3 has been connected with the basic body 2 in a sealed manner. The left end 5 of the balloon is connected with a separate end-section 8 at the same longitudinal position as the end of the basic body 2. In this end-section 8 a canal 9 has been formed to which for instance contrast medium can be supplied via a separate inner tube-like element 7 which has been received in the lumen 10 of the basic body 2. The inner tube-like element 7 is connected with the end-section 8 at the same height as the end 5 of the balloon member 3 and the end of the basic body 2.

In the illustrated example of the embodiment two groups of elongated openings 12 and 13 respectively, extending in a longitudinal direction, have been formed inside the section 6 of the basic body 2 enclosed by the balloon member 13. Of each group the openings 12 and 13 respectively have been arranged equally divided around the circumference, each time over the same longitudinal distance. Each time strip-shaped sections 16, which can bend outwards relatively easily, are defined in between the openings 12, 13, as can be seen in fig. 2. This bending outwards occurs when medium under pressure is supplied via the lumen 10 of the basic body 2. This medium flows via the openings 12, 13, into the balloon member 3 which consequently expands. On expansion the axial length reduces from the size indicated with number 14 in fig. 1 to the size indicated with number 15 in fig. 2. This reduction in length is possible as the strip-shaped sections bend outwards as can be seen clearly in fig. 2.

After allowing the medium under pressure to flow away, the catheter tip will resume the shape shown in fig. 1 as a result of the elasticity of the strip-shaped section 16.

The balloon-shaped member 3 has been made of a relatively inelastic material, so that the shape of the balloon-shaped member 3 in the expanded state is substantially predetermined. In the non-expanded state the material of which the balloon-shaped member has been made is folded in pleads against the basic body 2. This has not been illustrated in detail in fig. 1; however, this method of folding a balloon-shaped member is as such known from the current state of the technique. Because of the size of the openings, the material can be folded in such a way that the non-expanded diameter is relatively small.

## Claims

1. Catheter comprising a tube-like basic body (2) with a proximal and a distal end and with at least one lumen (10) defined by the wall of the basic body, wherein a balloon member (3) has been arranged at the distal end characterized in that the balloon (3), on both ends (4, 5) of an enclosed section of the basic body (2), is connected to the basic body (2) in a sealed manner and wherein, in the enclosed section a number of elongated openings (12, 13), extending in a longitudinal direction, have been formed in the wall of the basic body (2).

2. Catheter as claimed in claim 1, wherein the openings (12, 13) have been arranged over the same longitudinal distance and are equally divided around the circumference.

3. Catheter as claimed in claim 2, wherein the openings (12, 13) have been arranged in at least two, in a longitudinal direction, successive groups, wherein, in between adjoining groups, there is a section (6) of the basic body (2) which is free of openings.

4. Catheter as claimed in claim 2 or 3, wherein each group comprises three openings.

5. Catheter as claimed in claim 4, wherein each opening (12, 13) is wider than the material of the basic body (2) remaining in between adjoining openings (12, 13) of a group.

6. Catheter as claimed in one of the previous claims, wherein the distal end (5) of the basic body (2) and the relatively distal end of the balloon member are connected at the same longitudinal position with an end-section (8) of a catheter.

7. Catheter as claimed in claim 6, wherein a tubelike element (7) has been received inside the lumen (10) of which the distal end is connected with the end-section (8) at the same longitudinal position as the basic body (2).

8. Catheter as claimed in one of the previous claims, wherein the balloon member has been made of relatively inelastic material.

## Patentansprüche

1. Katheter, bestehend aus einem schlauchartigen Grundkörper (2) mit einem proximalen Ende und einem distalen Ende und mit wenigstens einem Lumen (10), das durch die Wand des Grundkörpers definiert ist, wobei ein Ballonkörper (3) am distalen Ende angeordnet ist,
dadurch gekennzeichnet, daß der Ballon (3) an beiden Enden (4, 5) eines umschlossenen Abschnitts des Grundkörpers (2) mit dem Grundkörper (2) dichtend verbunden ist, und daß der umschlossene Abschnitt eine Anzahl von in Längsrichtung verlaufenden langgestreckten Öffnungen (12, 13) aufweist, die in der Wand des Grundkörpers (2) ausgeformt sind.

2. Katheter nach Anspruch 1, bei welchem die Öffnungen (12, 13) sich über den gleichen Längsabstand erstrecken und im gleichen Winkelabstand über den Umfang angeordnet sind.

3. Katheter nach Anspruch 2, bei welchem die Öffnungen (12, 13) in wenigstens zwei in Längsrichtung verlaufenden Gruppen angeordnet sind, wobei zwischen den benachbarten Gruppen ein Abschnitt (6) des Grundkörpers (2) vorhanden ist, der frei von Öffnungen ist.

4. Katheter nach den Ansprüchen 2 oder 3, bei welchem jede Gruppe drei Öffnungen aufweist.

5. Katheter nach Anspruch 4, bei welchem jede Öffnung (12, 13) breiter ist als das Material des Grundkörpers (2), das zwischen benachbarten Öffnungen (12, 13) einer Gruppe verbleibt.

6. Katheter nach einem der vorhergehenden Ansprüche, bei welchem das distale Ende des Grundkörpers (2) und das jeweilige distale Ende des Ballonkörpers an der gleichen Längsposition wie ein Endabschnitt (8) des Katheters verbunden sind.

7. Katheter nach Anspruch 6, bei welchem ein schlauchartiges Element (7) innerhalb des Lumens (10) aufgenommen ist, dessen distales Ende mit dem Endabschnitt (8) an der gleichen Längsposition wie der Grundkörper (2) verbunden ist.

8. Katheter nach einem der vorhergehenden Ansprüche, bei welchem der Ballonkörper aus relativ unelastischem Material hergestellt ist.

## Revendications

1. Cathéter comprenant un corps de base (2) semblable à un tube, présentant une extrémité proximale et une extrémité distale et comportant au moins une lumière (10) définie par la paroi du corps de base, dans lequel un élément formant ballonnet (3) a été disposé à l'extrémité distale, caractérisé en ce que le ballonnet (3), aux deux extrémités (4, 5) d'une section recouverte du corps de base (2), est relié au corps de base (2) de façon étanche et dans lequel, dans la section recouverte, un certain nombre d'ouvertures allongées (12, 13), s'étendant dans une direction longitudinale, ont été ménagées dans la paroi du corps de base (2).

2. Cathéter selon la revendication 1, dans lequel les ouvertures (12, 13) ont été ménagées sur la même distance longitudinale et sont réparties de façon égale sur la circonférence.

3. Cathéter selon la revendication 2, dans lequel les ouvertures (12, 13) ont été ménagées dans au moins deux groupes, se suivant dans une direction longitudinale, dans lequel, entre des groupes contigus, se trouve une section (6) du corps de base (2) qui est dépourvue d'ouvertures.

4. Cathéter selon la revendication 2 ou 3, dans lequel chaque groupe comprend trois ouvertures.

5. Cathéter selon la revendication 4, dans lequel chaque ouverture (12, 13) est plus large que le matériau du corps de base (2) qui subsiste entre les ouvertures (12, 13) contigües d'un groupe.

6. Cathéter selon l'une des revendications précédentes, dans lequel l'extrémité distale (5) du corps de base (2) et l'extrémité relativement distale de l'élément formant ballonnet sont reliées, à la même position longitudinale, à une section terminale (8) d'un cathéter.

7. Cathéter selon la revendication 6, dans lequel un élément (7) semblable à un tube a été reçu à l'intérieur de la lumière (10) au niveau de laquelle l'extrémité distale est reliée à la section terminale (8), à la même position longitudinale que le corps de base (2).

8. Cathéter selon l'une des revendications précédentes, dans lequel l'élément formant ballonnet a été constitué dans un matériau relativement inélastique.
